(19) **Europäisches Patentamt**
**European Patent Office**
**Office européen des brevets**

(11) **EP 2 594 200 B1**

(12) **EUROPEAN PATENT SPECIFICATION**

(45) Date of publication and mention
of the grant of the patent:
**02.01.2019 Bulletin 2019/01**

(21) Application number: **10851579.2**

(22) Date of filing: **20.05.2010**

(51) Int Cl.:
***G16H 30/20*** (2018.01)

(86) International application number:
**PCT/CN2010/072969**

(87) International publication number:
**WO 2011/143820 (24.11.2011 Gazette 2011/47)**

(54) **METHOD FOR SIMULATING AND CORRECTING DEFORMATION OF CEREBRAL TISSUE IMAGES**

VERFAHREN ZUR SIMULATION UND KORREKTUR VON DEFORMATIONEN AUF HIRNGEWEBEBILDERN

PROCÉDÉ DE SIMULATION ET DE CORRECTION D'UNE DÉFORMATION D'IMAGES DE TISSU CÉRÉBRAL

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(43) Date of publication of application:
**22.05.2013 Bulletin 2013/21**

(73) Proprietor: **Fudan University**
**Shanghai 200433 (CN)**

(72) Inventors:
• **SONG, Zhijian**
**Shanghai 200032 (CN)**
• **WANG, Manning**
**Shanghai 200032 (CN)**
• **ZHANG, Chenxi**
**Shanghai 200032 (CN)**
• **LI, Wensheng**
**Shanghai 200032 (CN)**

• **YAO, Demin**
**Shanghai 200032 (CN)**

(74) Representative: **Lang, Christian et al**
**LangPatent Anwaltskanzlei**
**Ingolstädter Straße 5**
**80807 München (DE)**

(56) References cited:
**WO-A1-2006/028474     WO-A1-2006/118548**
**WO-A2-2005/122026     WO-A2-2008/063494**
**CN-A- 1 582 863       CN-A- 1 914 640**

• **DUMPURI ET AL: "An atlas-based method to compensate for brain shift: Preliminary results", MEDICAL IMAGE ANAL, OXFORD UNIVERSITY PRESS, OXOFRD, GB, vol. 11, no. 2, 8 March 2007 (2007-03-08), pages 128-145, XP005918528, ISSN: 1361-8415, DOI: 10.1016/J.MEDIA.2006.11.002**

EP 2 594 200 B1

**Description**

FIELD OF THE INVENTION

The invention belongs to the field of medical image processing and application and relates to a method for simulation of intra-operative brain deformation and correcting deformation of preoperative brain images. The invention specifically relates to a method for simulating intra-operative brain deformation and warping preoperative images during neurosurgery, which can be used for improvement of the navigation accuracy of image-guided neurosurgery system.

BACKGROUND OF THE INVENTION

Image Guided Neurosurgery (IGNS) systems which can provide accurate positioning information for neurosurgery have important implications for improvement of neurosurgical quality and reduction of surgical injury [1, 2]. The brain is not rigid and will shift over time during neurosurgery. Studies showed that cortical surface deformation was 5.14 $\pm$ 4.05 mm and tumor deformation was 3.53 $\pm$ 3.67 mm [3]. Tumor displacements can range up to 7.5 mm during tumor resection [4]. In consequence, preoperative images cannot account for intraoperative changes in brain morphology and the navigation accuracy of TGNS systems, therefore, deteriorates significantly.

Approaches for correcting brain deformation can be classified into two categories: intra-operative imaging and physical model methods. Intraoperative imaging, such as intra-operative MRI (iMRI) [5], can provides real-time brain images during neurosurgery, but its exorbitant cost restricts its application [6]. Biomechanical models, guided by intra-operative sparse information, can be used to estimate intraoperative brain-shift. Compared to intraoperative imaging techniques, the biomechanical-model-based approach is cost-effective and efficient.

The most commonly used biomechanical models are consolidation theory models and linear elastic models. Consolidation theory models treat the brain tissue as a porous solid tissue matrix infused with an interstitial fluid [7, 8], while linear elastic models regard the brain tissue as an homogeneous elastic material, in which the stress is a linear function of the strain [9, 10]. Although consolidation theory appears to be a more realistic description of the continuum than simple elasticity, they are computationally more expensive than linear elastic models [11]. Moreover, the boundary condition requirements of consolidation theory models can be challenging to achieve during neurosurgery [12]. Since craniotomy-induced brain shift is a relatively small deformation and a slow process, it is a good approximation to use a linear elastic model to model small and slow tissue deformation [6]. The linear elastic model, using intraoperative sparse information as boundary conditions, which includes real-time cortical surface details and can be acquired easily by a surface tracking algorithm, is suited to clinical practice for intraoperative brain-deformation correction. Technologies and references related to the invention are listed as following:

[1] M. Knauth, C. R. Wirtza, V. M. Tronniera, N. Arasa, S. Kunzea, and K. Sartora, "Intraoperative MR imaging increases the extent of tumor resection in patients with high-grade gliomas," American journal of neuroradiology, vol. 20, pp. 1642-1646 1999.
[2] F. A. Jolesz, "Image-guided procedures and the operating room of the future," Radiology, vol. 204, pp. 601-602, 1997.
[3] G. H. Du, L. F. Zhou, Y. Mao and J. S. Wu, "Intraoperative brain shift in neuronavigator-guided surgery," Chinese Journal of Minimally Invasive Neurosurgery, vol. 7, pp-65-68, 2007.
[4] R. D. Bucholz, D. D. Yeh, J. Trobaugh, L. L. MeDurmont, C. D. Sturm, C. Baumann, J. M. Henderson, A. Levy, and P. Kessman, "The correction of stereotactic inaccuracy caused by brain shift using an intraoperative ultrasound device," Lecture Notes In Computer Science, vol. 1225, pp. 459-466, 1997.
[5] A. Nabavi, P. M. Black, D. T. Gering, C.-F. Westin, V. Mehta, R. S. Pergolizzi, M. Ferrant, S. K. Warfield, N. Hata, R. B. Schwartz, W. M. W. III, R. Kikinis, and F. A. Jolesz, "Serial intraoperative magnetic resonance imaging of brain shift.," Neurosurgery vol. 48, pp. 787-797, 2001.
[6] O. Skrinjar, A. Nabavi, and J. Duncan, "Model-driven brain shift compensation," Medical image analysis vol. 6, pp. 361-373, 2002.
[7] M. I. Miga, K. D. Paulsen, F. E. Kennedy, J. Hoopes, A. Hartov, and D. W. Roberts, "Initial In-Vivo Analysis of 3d Heterogeneous Brain Computations for Model-Updated Image-Guided Neurosurgery." Lecture Notes in Computer Science, vol, 1496, pp. 743 -752, 1998.
[8] K. D. Paulsen, M. I. Miga, F. E. Kennedy, P. J. Hoopes, A. Hartov, and D. W. Roberts, "A computational model for tracking subsurface tissue deformation during stereotactic neurosurgery," IEEE transactions on bio-medical engineering vol. 46, pp. 213-225, 1999.
[9] D. Terzopoulos, J. Platt, A. Barr, and K. Fleischer, "Elastically deformable models," Computer Graphics, vol. 21, pp. 206-214, 1987.
[10] O. M. Skrinjar, C. Studholme, A. Nabavi, and J.S. Duncan, "Steps toward a stereo-camera-guided biomechanical model for brain shift compensation," Lecture Notes In Computer Science, vol. 2082, 2001.

[11] T. K. Sinha, B. M. Dawant, V. Duay, D. M. Cash, R. J. Weil, R.C. Thomson, K. D. Weaver, and M. I. Miga, "An atlas-based method to compensate for brain-shift: Preliminary results" Medical Image Analysis, vol. 11, pp. 128-145, 2007,

[12] M. I. Miga, K. D. Paulsen, J. M. Lemery, S. D. Eisner, A. Hartov, F. E. Kennedy, and D. W. Roberts, "Model-updated image guidance: initial clinical experiences with gravity-induced brain deformation," IEEE transactions on medical imaging vol. 18, pp. 866-874, 1999.

[13] D. L. G. Hill, C. R. Maurer, M. Y. Wang, R. J. Maciunas, J. A. Barwise, and J. M. Fitzpatrick, "Estimation of intraoperative brain surface movement" Lecture Notes In Computer Science, vol, 1205, pp.449-458 1997

[14] M. A. Audette, K. Siddiqi, F.P. Ferrie, and T. M. Peters, "An integrated range-sensing, segmentation and registration framework for the characterization of intra-surgical brain deformations in image-guided surgery," Computer Vision and Image Understanding, vol. 89, pp. 226-251, 2002.

WO 2008/063494 A2 relates to an apparatus and methods of compensating for organ deformation, as best suited for compensation of deformation of a liver, in contrast to deformation of a brain where the cranium is providing for confines. WO 2008/063494 A2 specifically teaches to use finite element models. The publication from DUMPURI ET AL: "An atlas-based method to compensate for brain shift: Preliminary results" (in MEDICAL IMAGE ANAL, OXFORD UNIVERSITY PRESS, OXOFRD, GB, vol. 11, no. 2 (2007), pages 128-145) further refers to a conventional method to compensate for brain shift, in which an atlas of model deformations based on the complex loading conditions is computed preoperatively and used with a constrained linear inverse model approach to predict the intraoperative brain shift. In more detail, it teaches a method for compensating intraoperative brain shift using a computational model, in which a constrained linear inverse model in combination with a biomechanical tissue model best fitted the measured intraoperative data, wherein the model is driven by brain deformations.

SUMMARY OF THE INVENTION

The invention aims to provide a method of simulation and intraoperative brain deformation and correction of deformation of preoperative brain images. The method can be easily used for intraoperative brain-deformation correction with real-time performance and high accuracy in IGNS systems and improve navigation accuracy and convenience, and clinical utility of IGNS systems in clinical practice.

The invention uses a biomechanical model based on linear elasticity theory to simulate brain-shift behavior. A laser range scanner (LRS), capable of generating textured point-clouds, is used to track exposed cortical surface deformations, which are then used to calculate the nodal displacements of the exposed brain surface that serve as boundary conditions to the linear elastic finite element (FE) model to infer a volumetric deformation field. Then, the partial differential equations (PDEs) of the linear elastic model can be solved using a finite element method with constraint of the previously defined boundary conditions. Finally, the warped preoperative images are obtained through a back-interpolation method and transferred to an IGNS system and used to provide intraoperative navigation.

The technical proposals of this invention are given below. First, the brain is extracted out of the skull using a Level set algorithm based automatic segmentation method. Second, a patient-specific multi-resolution mesh is then generated from the segmented brain parenchyma. Next, a LRS, combined with an optical tracking system and a surface-tracking algorithm, is used to estimate the nodal displacements of the exposed brain surface, which are then imposed as boundary conditions to the linear elastic FE model to infer a volumetric deformation field. Then, the partial differential equations (PDEs) of the linear elastic model are solved using a finite element method with constraint of the boundary conditions. A back-interpolation method is then employed to warp preoperative images and a ray casting is used to visualize them. Finally, the warped preoperative images are transferred to an IGNS system and used to provide intraoperative navigation.

The method for simulating intraoperative brain deformation and correcting deformation of preoperative brain images comprises the following steps.

1. Prior to finite element mesh generation, an automatic segmentation method based on Level Set algorithm is used to extract the brain out of the preoperative MR images. The segmentation method includes two steps. First, a coarse segmentation of the brain tissue is performed using a combination of some simple image processing operators, including the thresholds, the erosion, the region growing and the dilation algorithm. Then, the segmentation is refined with Level Set algorithm.

2. A patient-specific multi-resolution mesh is generated from the segmented brain parenchyma. A finer tetrahedral mesh is used close to the cortical surface, elsewhere the mesh size is larger. A mesh is a discretization of a geometric domain into small simple shapes, called elements, which are connected by nodes. Since brain shift is larger at brain surface than shift inside the brain, a multi-resolution mesh is used to discretize the segmented brain in order to exactly simulate the brain surface deformation. The mesh density is high at the boundary and low in the interior. Tetrahedron elements are employed instead of hexahedron elements in order to more precisely represent the brain surface. The multi-resolution mesh algorithm combines an Octree algorithm with a marching tetrahedron (MT) algorithm. To improve the computational efficiency, this algorithm uses the case table to record all cases of clipping. The algorithm is described as:

(1) Use a Octree algorithm to separate the 3D image space into even hexahedron mesh.
(2) Divide each hexahedron into five tetrahedrons.
(3) Subdivide the tetrahedrons at the boundary to obtain the multi-resolution mesh.
(4) Use a quasi MT algorithm to clip the mesh from the background and then acquire multi-resolution mesh of the brain. During computation, the case table is used to improve the efficiency.

3. Biomechanical properties are assigned to each element and a final linear elastic FE model can be created. The linear elastic model regards the brain tissue as an homogeneous elastic material, in which the stress is a linear function of the strain. The linear elastic model can be expressed as a series of partial differential equations (PDEs) and is described as the following formula:

$$\nabla^2 \mu_x + \frac{1}{1-2v} \frac{\partial}{\partial x} \left( \frac{\partial \mu_x}{\partial x} + \frac{\partial u_y}{\partial y} + \frac{\partial u_z}{\partial z} \right) + \frac{F_x}{\mu} = 0 \qquad (1)$$

$$\nabla^2 \mu_y + \frac{1}{1-2v} \frac{\partial}{\partial y} \left( \frac{\partial \mu_x}{\partial x} + \frac{\partial u_y}{\partial y} + \frac{\partial u_z}{\partial z} \right) + \frac{F_y}{\mu} = 0 \qquad (2)$$

$$\nabla^2 \mu_z + \frac{1}{1-2v} \frac{\partial}{\partial z} \left( \frac{\partial \mu_x}{\partial x} + \frac{\partial u_y}{\partial y} + \frac{\partial u_z}{\partial z} \right) + \frac{F_z}{\mu} = 0 \qquad (3)$$

where $u$ is the displacement vector, $F$ is the force vector and $v$ is Poisson's ratio. $\mu$ is defined as: $\mu = E/2(1+v)$, where $E$ is Young's modulus.

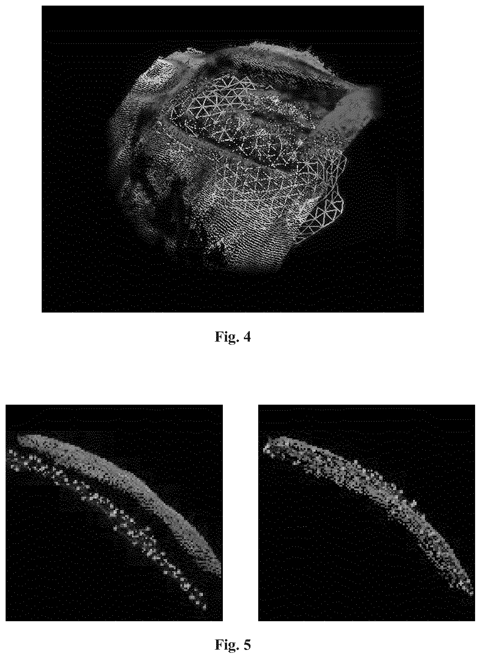

After the linear elastic FE model is created, a surface tracking algorithm combined with a LRS and an optical tracking system is used to calculate the nodal displacements of the exposed cortical surface, which is an alternative to the forces that produce brain shift, to constrain the FE equations as boundary conditions to infer a volumetric deformation field. Prior to tracking of the moving of the surface, the initial surface (image space), which is acquired by extracting from mesh and the deformed surface (LRS space), which is acquired by scanning the exposed cortical surface using the LRS, are needed to transform into the same space. This process requires space coordinate transformation between the four spaces: (1) coordinate transformation from LRS space to the tracked tool space; (2) coordinate transformation from the tracked tool space to the tracking system space; (3) coordinate transformation from the tracking system space to the reference frame space; (4) coordinate transformation from the reference frame space to the image space. The tracked tool is fixed on the LRS and can be tracked by the optical tracking system. The reference frame is fixed onto the patient's and can be also tracked by the optical tracking system. During surgery, the first coordinate transformation is acquired in the module of calibration in brain deformation system (BDS); the second, third and fourth coordinate transformations are acquired in IGNS and transferred to BDS through the module of communication. As the four transformations are available, the deformed surface (LRS space) is registered to the initial surface (image space), as shown in Fig.4. The surface deformations are then computed using a robust point matching (RPM) algorithm.

4. The point cloud of the deformed surface is captured by scanning the exposed cortical surface using a LRS, which is capable of generating textured point-clouds. Then the nodal displacements of the exposed cortical surface can be calculated using a surface tracking algorithm and used as boundary conditions of the FE equations to infer a volumetric deformation field;

Based on the proposal described above, the step 4 is used to track exposed cortical surface deformations by means of minimization of the energy function:

$$\min_{C,f} E(C,f) = \min_{C,f} \sum_{j=1}^{N} \sum_{i=1}^{M} c_{ij} \left\| y_j - f(x_i) \right\|^2 + \lambda \iint \left[ \left( \frac{\partial^2 f}{\partial x^2} \right)^2 + 2 \left( \frac{\partial^2 f}{\partial x \partial y} \right)^2 + \left( \frac{\partial^2 f}{\partial y^2} \right)^2 \right]$$

where $X=\{x, i=1,2, ...M\}$, is the point set of mesh surface (initial surface), $Y=\{y,j=1,2, ...N\}$, is the point set of point cloud (deformed surface), $C$ or $\{c_{ij}\}$ is the correspondence matrix and used to measure the correspondence between $x_i$, and $y_j$, and $\lambda$ is the weight parameters that balance the second terms. The non-rigid transformation $f$, which is used to map $x_i$ to $y_j$, can be solved using the thin-plate spline (TPS), and then exposed surface deformation $a_0$ is acquired.

5. The acquired boundary conditions are imposed to the FE equations. The brain model is first discretized using tetrahedron elements in order to solve the PDEs. Then the acquired boundary conditions are used to constraint the FE equations to infer the deformation of the entire brain-After discretization of the brain model, the PDEs can be changed to System of equations by FEM:

$$Ka = P$$

where $K$ is structure rigid matrix, $P$ is structure load vector and $a$ is node displacement vector. $K$ can be determined by two parameters: $E$ (Young's modulus) and $v$ (Poisson's ratio).

The Portable, Extensible Toolkit for Scientific Computation (PETSc) package is utilized to parallelize the solving of linear matrix systems in order to improve computational speed.

6. A back-interpolation method is then employed to warp preoperative images and a ray casting is used to visualize them. The back-interpolation method is described as:

(1) Find all integer grid points for each element in the deformed mesh
(2) Compute its position $P_1$ in pre-operative image for each integer grid point with a shape function
(3) Get its intensity by tri-linear interpolation at position $P_1$ in pre-operative image.
(4) Render warped image using ray casting.

The above-described method for simulating intraoperative brain deformation and correcting deformation of preoperative brain images has the advantages that:

(1) An automatic segmentation method based on Level Set algorithm is employed, which makes it possible to extract the brain out of the preoperative MR images more accurately and subtly.
(2) A TPS based surface-tracking algorithm combined with a LRS and an optical tracking system is proposed, which can guarantee one-to-one correspondence between feature points in point cloud and those in preoperative images. The algorithm is more efficient and robust
(3) A method of multi-processor based parallel computing is used to solve the FE equations, which is computationally more efficient and can help to make a quick update of preoperative images and enhance the real-time performance of surgical navigation.

BRIEF DESCRIPTION OF THE DRAWINGS

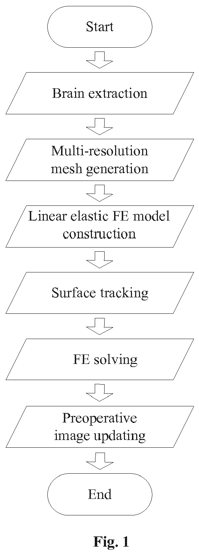

Fig. 1 is a flowchart for correction of intra-operative brain deformation.

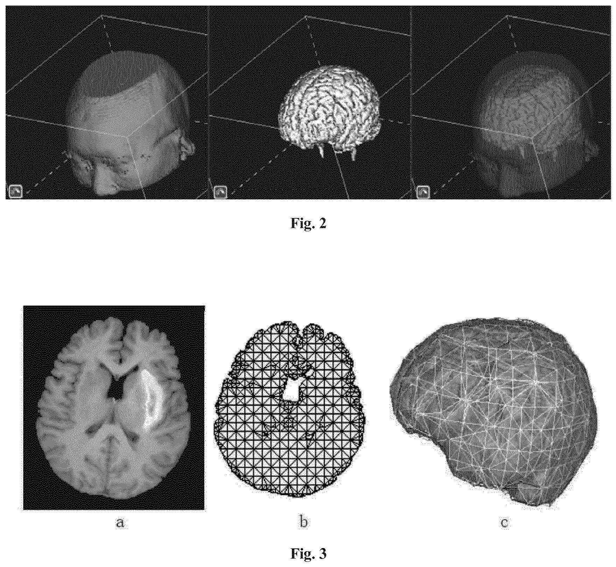

Fig. 2 shows the 3D results of the brain segmentation based on 256 x 256 x 48 MRI, according to the automatic segmentation method based on Level Set algorithm.
Fig. 3 shows the result of the multi-resolution mesh based on 256 x 256 x 48 MRI: (a) a preoperative MRI axial slice, (b)a mesh corresponding to (a), and (c)3D multi-resolution mesh.
Fig. 4 shows the initial surface and the deformed surface in the same image space after rigid registration.
Fig. 5 shows the result of surface tracking where (a) shows that the initial surface has sunk from the deformed surface and (b) shows that the initial surface coincides with the deformed surface after surface tracking.

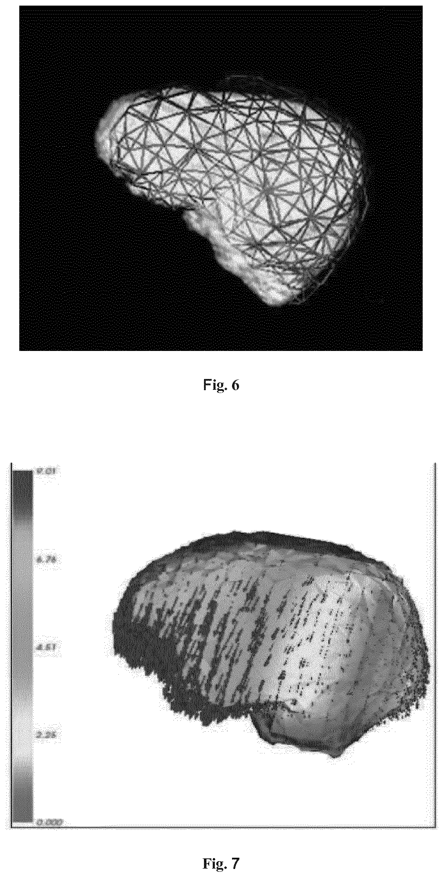

Fig. 6 shows an overlay of the undeformed brain (grey color) and the deformed mesh (red-color).
Fig. 7 shows the 3D visualization of the deformation field (the arrows show the direction of the deformation and the color shows the magnitude of the deformation which changes from small to large as the color changes from red to blue)

DETAILED DESCRIPTION OF EMBODIMENTS

Embodiment 1

1. A brain was extracted out of preoperative MR images (256 x 256 x 48) using an automatic segmentation method based on Level Set algorithm.

2. Using a multi-resolution mesh algorithm, a multi-resolution mesh was then generated from the segmented brain parenchyma. The mesh contained 5410 nodes and 18485 elements. The size of maximum tetrahedron at brain surface was 7.5 mm x 7.5 mm x 7.5 mm.

3. Biomechanical properties were assigned to each element of the linear elastic brain model. The Young's modulus (E) of the brain tissue was set 3000 Pa and Poisson's ratio ($v$) was set 0.45 [12] for the FE equations to be solved.

4. After the craniotomy and duratomy, a LRS was used to acquire range data of the deformed cortical surface. The acquisition camera of the LRS was about 450 mm from the exposed cortical surface in a normal direction. It took 5-7 seconds to acquire the range data with point set density of 512 x 512.

5. The nodes on the constructed FE mesh surface were extracted and defined as the initial surface, i.e., undeformed surface, and the deformed surface (LRS space) was then registered to the initial surface (image space). This process required a fiducial-based registration between image and patient spaces, calibration of the LRS, and tracking the patient and LRS throughout the LRS scan. Through calibration of the LRS, a transformation from LRS space to the tracked tool space can be acquired. A Polaris optical tracking system was used to track a track tool and a reference frame, and then obtain a transformation from the tracked tool space to the Polaris optical tracking system space and a transformation from the Polaris optical tracking system space to the reference frame space. As the transformations needed and the point clouds of the deformed surface were available, the nodal displacements of the exposed cortical surface can be calculated and imposed as boundary conditions to the FE equations.

6. The linear elastic model is described as a series of PDEs which can be changed to system of equations by FEM: $Au = b$ (where $A$ is structure rigid matrix, $b$ is structure load vector and $u$ is node displacement vector to be calculated). Conjugate graduate (CG) was used to solve the linear system of equations which contained 16230 equations (i.e. 5410 nodes x 3 dimensions). With the constraint of the boundary conditions, the singularity of matrix A was eliminated and displacement vector $u$ was calculated. As displacement of each node within the FE mesh was available, full-volume displacement fields can be obtained with the aid of shape fonction.

7. A back-interpolation method was finally employed to warp preoperative images: (1) Find all integer grid points for each element in the deformed mesh; (2) Compute its position $P_1$ in pre-operative image for each integer grid point with a shape function; (3) Get its intensity by tri-linear interpolation at position $P_1$ in pre-operative image; (4) Render warped image using ray casting. The warped images were used to guide surgery.

8. It took 2 minutes to correct intra-operative brain deformation on a workstation (Pentium IV2.6 G, 2 GB RAM) upon Windows XP platform.

## Claims

1. A method for simulating intraoperative brain deformation and correcting deformation of preoperative brain images, based
on a linear elastic model, the model being driven by exposed cortical surface deformations, the exposed cortical surface deformations being tracked by a laser-range scanner (LRS) and an optical tracking system, the LRS being capable of generating textured point-clouds, the method comprising:

(1) using a linear elastic model to simulate craniotomy-induced brain deformation;
(2) calculating the nodal displacements of the exposed cortical surface which are then imposed as boundary conditions to the linear elastic model to infer a volumetric deformation field;
(3) solving the partial differential equations (PDEs) of the linear elastic model using a finite element method with constraint of the boundary conditions;
(4) warping preoperative images using a back-interpolation method and visualizing them using ray casting.

2. The method according to claim 1, wherein the PDEs of the linear elastic model of step (3) are:

$$\nabla^2 \mu_x + \frac{1}{1-2v} \frac{\partial}{\partial x} \left( \frac{\partial \mu_x}{\partial x} + \frac{\partial \mu_y}{\partial y} + \frac{\partial \mu_z}{\partial z} \right) + \frac{F_x}{\mu} = 0$$

$$\nabla^2 \mu_y + \frac{1}{1-2v} \frac{\partial}{\partial y} \left( \frac{\partial \mu_x}{\partial x} + \frac{\partial \mu_y}{\partial y} + \frac{\partial \mu_z}{\partial z} \right) + \frac{F_y}{\mu} = 0$$

$$\nabla^2 \mu_z + \frac{1}{1-2v} \frac{\partial}{\partial z} \left( \frac{\partial \mu_x}{\partial x} + \frac{\partial \mu_y}{\partial y} + \frac{\partial \mu_z}{\partial z} \right) + \frac{F_z}{\mu} = 0$$

where $\mu$ is the displacement vector, F is the force vector and $v$ is Poisson's ratio. $\mu$ is defined as: $\mu = E/2\,(1+v)$, where $E$ is Young's modulus.

3. The method according to claim 1, wherein the step (2) of calculating the boundary conditions comprises the steps of:

(1) capturing the deformed surface in the craniotomy area by LRS scanning;
(2) transforming the deformed surface (LRS space) into the image space using an optical tracking system;
(3) constructing minimization of the energy function to calculate the nodal displacements of the exposed cortical surface.

4. The method according to claim 3, wherein the step of transforming the deformed surface (LRS space) into the image space comprises the steps of:

(1) transforming LRS space into the tracked tool space;
(2) transforming the tracked tool space into the optical tracking system space;
(3) transforming the optical tracking system space into the reference frame space;
(4) transforming the reference frame space into the preoperatively-acquired image space (initial surface space).

5. The method according to claim 3, wherein the step of calculating cortical surface deformations by means of minimization of the energy function:

$$\min_{C,f} E(C,f) =$$

$$\min_{C,f} \sum_{j=1}^{N} \sum_{i=1}^{M} c_{ij} \, \|y_j - f(x_i)\|^2 + \lambda \iint \left[ \left( \frac{\partial^2 f}{\partial x^2} \right)^2 + \left( \frac{\partial^2 f}{\partial y^2} \right)^2 + \left( \frac{\partial^2 f}{\partial z^2} \right)^2 \right]$$

where $X = \{x_i, i = 1,2, \dots M\}$, is the point set of mesh surface (initial surface),
$Y = \{y_j, j = 1,2, \dots N\}$, is the point set of point cloud (deformed surface), C or $\{c_{ij}\}$ is the correspondence matrix and used to measure the correspondence between $x_i$ and $y_j$ and $\lambda$ is the weight parameters that balance the second terms, and wherein the non-rigid transformation $f$, which is used to map $x_i$ and $y_j$, can be solved using the thin-plate spline (TPS), and then surface deformation $a_0$ is acquired.

6. The method according to claim 4, wherein the track tool is fixed on the LRS and can be tracked by the optical tracking system, and wherein the reference frame is fixed onto the patient's head and can be also tracked by the optical tracking system.

**Patentansprüche**

1. Verfahren zur Simulation intraoperativer Gehirndeformation und Korrektur der Deformation präoperativer Gehirnaufnahmen, basierend
auf einem linear elastischen Modell, wobei das Modell durch exponierte kortikale Oberflächendeformationen getrieben wird, wobei die exponierten kortikalen Oberflächendeformationen von einem Laser-Range-Scanner (LRS) und einem optischen Überwachungssystem verfolgt werden, wobei der LRS in der Lage ist strukturierte Punktwolken zu erzeugen, wobei das Verfahren umfasst:

(1) Verwenden eines linear elastischen Modells zum Simulieren Kraniotomieinduzierter Gehirndeformation;
(2) Berechnen von Knotenverschiebungen der exponierten kortikalen Oberfläche, die dann die Randbedingungen für das lineare elastische Modell festlegen, um auf ein volumetrisches Deformationsfeld zu schließen;
(3) Lösen der partiellen Differenzialgleichungen (PDEs) des linearen elastischen Modells unter Verwendung einer Finite-Elemente-Methode mit Beschränkung durch die Randbedingungen;
(4) Verziehen präoperativer Bilder mit einem Rück-Interpolationsverfahren und Visualisieren dieser mittels Raycsting.

2. Verfahren nach Anspruch 1, bei welchem die PDEs des linearen elastischen Modells aus Schritt 3 sind:

$$\nabla^2 \mu_x + \frac{1}{1-2v}\frac{\partial}{\partial x}\left(\frac{\partial \mu_x}{\partial x} + \frac{\partial \mu_y}{\partial y} + \frac{\partial \mu_z}{\partial z}\right) + \frac{F_x}{\mu} = 0$$

$$\nabla^2 \mu_y + \frac{1}{1-2v}\frac{\partial}{\partial y}\left(\frac{\partial \mu_x}{\partial x} + \frac{\partial \mu_y}{\partial y} + \frac{\partial \mu_z}{\partial z}\right) + \frac{F_y}{\mu} = 0$$

$$\nabla^2 \mu_z + \frac{1}{1-2v}\frac{\partial}{\partial z}\left(\frac{\partial \mu_x}{\partial x} + \frac{\partial \mu_y}{\partial y} + \frac{\partial \mu_z}{\partial z}\right) + \frac{F_z}{\mu} = 0$$

wobei $\mu$ der Verschiebungsvektor, F der Kraftvektor und *v* die Poissonsche Konstante ist und $\mu$ definiert ist als: $\mu$ = *E*/2 (1 + *v*), wobei *E* der Elastizitätsmodul ist.

3. Verfahren nach Anspruch 1, bei welchem Schritt 2 zum Berechnen der Randbedingungen die Schritte umfasst:

(1) Erfassen der verformten Oberfläche in dem Kraniotomie-Bereich mittels LRS-Scanning;
(2) Transformieren der deformierten Oberfläche (LRS Raum) in den Bildraum mit einem optischen Tracking-System;
(3) Konstruieren einer Minimierung der Energiefunktion, um die Knotenverschiebungen der exponierten kortikalen Oberfläche zu berechnen.

4. Verfahren nach Anspruch 3, bei welchem der Schritt des Transformierens der deformierten Oberfläche (LRS Raum) in den Bildraum die Schritte umfasst:

(1) Transformieren des LRS-Raums in den erfassten Werkzeugraum;
(2) Transformieren des erfassten Werkzeugraums in den optischen Überwachungsraum;
(3) Transformieren des optischen Überwachungsraums in den Referenzrahmenraum;
(4) Transformieren des Referenzrahmenraums in den präoperativ erworbenen Bildraum (ursprünglicher Oberflächenraum).

5. Verfahren nach Anspruch 3, bei welchem der Schritt der Berechnung kortikaler Oberflächendeformationen mittels Minimierung der Energiefunktion erfolgt

$$\min_{C,f} E(C,f) =$$

$$\min_{C,f} \sum_{j=1}^{N} \sum_{i=1}^{M} c_{ij} \|y_j - f(x_i)\|^2 + \lambda \iint \left[\left(\frac{\partial^2 f}{\partial x^2}\right)^2 + \left(\frac{\partial^2 f}{\partial y^2}\right)^2 + \left(\frac{\partial^2 f}{\partial z^2}\right)^2\right]$$

wobei $X = \{x_i, i = 1,2, ... M\}$, die Punktmenge der Netzoberfläche ist (ursprüngliche Oberfläche), $Y = \{y_j, j = 1,2, ...$

*N}*, die Punktmenge der Punktwolke (deformierte Oberfläche) ist, C oder {$c_{ij}$} die Korrespondenz-Matrix ist und der Messung der Korrespondenz zwischen $x_i$ und $y_j$ dient und λ der Gewichtsparameter ist, der die zweite Terme ausgleicht, und wobei die nicht starre Transformation *f*, die verwendet wird, um $x_i$ und $y_j$, abzubilden, unter Verwendung der Thin-Plate-Splines (TPS) gelöst werden kann, und dann die Oberflächenverformung $a_0$ erfasst wird.

**6.** Verfahren nach Anspruch 3, bei welchem das Track-Tool am LRS fixiert ist und vom optischen Tracking-System nachverfolgt werden kann, und wobei der Bezugsrahmen auf dem Kopf des Patienten befestigt ist und auch vom optischen Tracking-System verfolgt werden kann.

**Revendications**

**1.** Méthode pour simuler une déformation du cerveau intra-opératoire et corriger une déformation d'images du cerveau peropératoire,
en fonction d'un modèle élastique linéaire, le modèle étant commandé par des déformations de surface corticale exposée, les déformations de surface corticale exposée étant suivies par un scanner laser (LRS) et un système de poursuite optique, le LRS étant capable de générer des nuages de points texturés, la méthode comprenant :

(1) l'utilisation d'un modèle élastique linéaire pour simuler une déformation du cerveau induite par craniotomie;
(2) le calcul des déplacements nodaux de la surface corticale exposée qui sont alors imposés en tant que conditions limites au modèle élastique linéaire pour inférer un champ de déformation volumétrique;
(3) la résolution des équations aux dérivées partielles (PDE) du modèle élastique linéaire à l'aide d'une méthode des éléments finis avec une contrainte des conditions limites;
(4) le gauchissement d'images préopératoires à l'aide d'une méthode d'interpolation arrière et leur visualisation à l'aide d'un lancer de rayons.

**2.** Méthode selon la revendication 1, dans laquelle les PDE du modèle élastique linéaire de l'étape (3) sont:

$$\nabla^2 \mu_x + \frac{1}{1-2v}\frac{\partial}{\partial x}\left(\frac{\partial \mu_x}{\partial x} + \frac{\partial \mu_y}{\partial y} + \frac{\partial \mu_z}{\partial z}\right) + \frac{F_x}{\mu} = 0$$

$$\nabla^2 \mu_y + \frac{1}{1-2v}\frac{\partial}{\partial y}\left(\frac{\partial \mu_x}{\partial x} + \frac{\partial \mu_y}{\partial y} + \frac{\partial \mu_z}{\partial z}\right) + \frac{F_y}{\mu} = 0$$

$$\nabla^2 \mu_z + \frac{1}{1-2v}\frac{\partial}{\partial z}\left(\frac{\partial \mu_x}{\partial x} + \frac{\partial \mu_y}{\partial y} + \frac{\partial \mu_z}{\partial z}\right) + \frac{F_z}{\mu} = 0$$

où $\mu$ est le vecteur de déplacement, *F* est le vecteur de force et *v* est le coefficient de Poisson, $\mu$ étant défini comme :
$\mu = E/2 (1 + v)$, où *E* est le module de Young.

**3.** Méthode selon la revendication 1, dans laquelle l'étape (2) de calcul des conditions limites comprend les étapes:

(1) de capture de la surface déformée dans la zone de craniotomie par balayage LRS;
(2) de transformation de la surface déformée (espace LRS) en l'espace d'image à l'aide d'un système de poursuite optique;
(3) de construction d'une minimisation de la fonction énergie pour calculer les déplacements nodaux de la surface corticale exposée.

**4.** Méthode selon la revendication 3, dans laquelle l'étape de transformation de la surface déformée (espace LRS) en l'espace d'image comprend les étapes:

(1) de transformation de l'espace LRS en l'espace d'outil suivi;
(2) de transformation de l'espace d'outil suivi en l'espace de système de poursuite optique;

(3) de transformation de l'espace de système de poursuite optique en l'espace de repère;
(4) de transformation de l'espace de repère en l'espace d'image acquise de façon préopératoire (espace de surface initiale).

**5.** Méthode selon la revendication 3, dans laquelle l'étape de calcul de déformations de surface corticale au moyen d'une minimisation de la fonction énergie:

$$\min_{C,f} E(C,f) =$$

$$\min_{C,f} \sum_{j=1}^{N} \sum_{i=1}^{M} c_{ij} \; \|y_j - f(x_i)\|^2 + \lambda \iint \left[ \left(\frac{\partial^2 f}{\partial x^2}\right)^2 + \left(\frac{\partial^2 f}{\partial y^2}\right)^2 + \left(\frac{\partial^2 f}{\partial z^2}\right)^2 \right]$$

où $X = \{x_i, i = 1, 2, ... M\}$, est l'ensemble de point de la surface de maille (surface initiale), $Y = \{y_j, j = 1, 2, ... N\}$, est l'ensemble de point du nuage de points (surface déformée), C ou $\{c_{ij}\}$ est la matrice de correspondance et est utilisé pour mesurer la correspondance entre $x_i$ et $y_j$ et $\lambda$ représente les paramètres de pondération qui équilibrent les seconds termes, et dans lequel la transformation non rigide $f$, qui est utilisée pour faire correspondre $x_i$ et $y_j$, peut être résolue à l'aide de la spline de plaque mince (TPS), puis une déformation de surface $a_0$ est acquise.

**6.** Méthode selon la revendication 4, dans laquelle l'outil de poursuite est fixé sur le LRS et peut être suivi par le système de poursuite optique, et dans laquelle le repère est fixé sur la tête du patient et peut également être suivi par le système de poursuite optique.

Start

Brain extraction

Multi-resolution
mesh generation

Linear elastic FE model
construction

Surface tracking

FE solving

Preoperative
image updating

End

**Fig. 1**

Fig. 2

a                    b                    c

Fig. 3

Fig. 4

Fig. 5

Fig. 6

Fig. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2008063494 A2

### Non-patent literature cited in the description

- **M. KNAUTH ; C. R. WIRTZA ; V. M. TRONNIERA ; N. ARASA ; S. KUNZEA ; K. SARTORA.** Intraoperative MR imaging increases the extent of tumor resection in patients with high-grade gliomas. *American journal of neuroradiology,* 1999, vol. 20, 1642-1646
- **F. A. JOLESZ.** Image-guided procedures and the operating room of the future. *Radiology,* 1997, vol. 204, 601-602
- **G. H. DU ; L. F. ZHOU ; Y. MAO ; J. S. WU.** Intraoperative brain shift in neuronavigator-guided surgery. *Chinese Journal of Minimally Invasive Neurosurgery,* 2007, vol. 7, 65-68
- **R. D. BUCHOLZ ; D. D. YEH ; J. TROBAUGH ; L. L. MEDURMONT ; C. D. STURM ; C. BAUMANN ; J. M. HENDERSON ; A. LEVY ; P. KESSMAN.** The correction of stereotactic inaccuracy caused by brain shift using an intraoperative ultrasound device. *Lecture Notes In Computer Science,* 1997, vol. 1225, 459-466
- **A. NABAVI ; P. M. BLACK ; D. T. GERING ; C.-F. WESTIN ; V. MEHTA ; R. S. PERGOLIZZI ; M. FERRANT ; S. K. WARFIELD ; N. HATA ; R. B. SCHWARTZ.** Serial intraoperative magnetic resonance imaging of brain shift. *Neurosurgery,* 2001, vol. 48, 787-797
- **O. SKRINJAR ; A. NABAVI ; J. DUNCAN.** Model-driven brain shift compensation. *Medical image analysis,* 2002, vol. 6, 361-373
- **M. I. MIGA ; K. D. PAULSEN ; F. E. KENNEDY ; J. HOOPES ; A. HARTOV ; D. W. ROBERTS.** Initial In-Vivo Analysis of 3d Heterogeneous Brain Computations for Model-Updated Image-Guided Neurosurgery. *Lecture Notes in Computer Science,* 1998, vol. 1496, 743-752

- **K. D. PAULSEN ; M. I. MIGA ; F. E. KENNEDY ; P. J. HOOPES ; A. HARTOV ; D. W. ROBERTS.** A computational model for tracking subsurface tissue deformation during stereotactic neurosurgery. *IEEE transactions on bio-medical engineering,* 1999, vol. 46, 213-225
- **D. TERZOPOULOS ; J. PLATT ; A. BARR ; K. FLEISCHER.** Elastically deformable models. *Computer Graphics,* 1987, vol. 21, 206-214
- **O. M. SKRINJAR ; C. STUDHOLME ; A. NABAVI ; J.S. DUNCAN.** Steps toward a stereo-camera-guided biomechanical model for brain shift compensation. *Lecture Notes In Computer Science,* 2001, vol. 2082
- **T. K. SINHA ; B. M. DAWANT ; V. DUAY ; D. M. CASH ; R. J. WEIL ; R.C. THOMSON ; K. D. WEAVER ; M. I. MIGA.** An atlas-based method to compensate for brain-shift: Preliminary results. *Medical Image Analysis,* 2007, vol. 11, 128-145
- **M. I. MIGA ; K. D. PAULSEN ; J. M. LEMERY ; S. D. EISNER ; A. HARTOV ; F. E. KENNEDY ; D. W. ROBERTS.** Model-updated image guidance: initial clinical experiences with gravity-induced brain deformation. *IEEE transactions on medical imaging,* 1999, vol. 18, 866-874
- **D. L. G. HILL ; C. R. MAURER ; M. Y. WANG ; R. J. MACIUNAS ; J. A. BARWISE ; J. M. FITZPATRICK.** Estimation of intraoperative brain surface movement. *Lecture Notes In Computer Science,* 1997, vol. 1205, 449-458
- **M. A. AUDETTE ; K. SIDDIQI ; F.P. FERRIE ; T. M. PETERS.** An integrated range-sensing, segmentation and registration framework for the characterization of intra-surgical brain deformations in image-guided surgery. *Computer Vision and Image Understanding,* 2002, vol. 89, 226-251
- An atlas-based method to compensate for brain shift: Preliminary results. **DUMPURI et al.** MEDICAL IMAGE ANAL. OXFORD UNIVERSITY PRESS, 2007, vol. 11, 128-145